## Europäisches Patentamt
## European Patent Office
## Office européen des brevets

(11) Veröffentlichungsnummer: **0 157 888**
**B1**

(12) # EUROPÄISCHE PATENTSCHRIFT

(45) Veröffentlichungstag der Patentschrift:
02.09.87

(51) Int. Cl.⁴: **A 61 B 1/30**

(21) Anmeldenummer: **84103223.8**

(22) Anmeldetag: **23.03.84**

(54) Einhand - Hysteroskop.

(43) Veröffentlichungstag der Anmeldung:
16.10.85 Patentblatt 85/42

(45) Bekanntmachung des Hinweises auf die Patenterteilung:
02.09.87 Patentblatt 87/36

(84) Benannte Vertragsstaaten:
AT BE CH DE FR GB IT LI NL SE

(56) Entgegenhaltungen:
DE-C-509 296
FR-A-2 520 223
US-A-3 368 552

(73) Patentinhaber: **Storz, Karl, Auf dem Schildrain 39, D-7200 Tuttlingen (DE)**

(72) Erfinder: **Der Erfinder hat auf seine Nennung verzichtet**

(74) Vertreter: **Wenzel, Joachim, Dipl.- Ing., Hauptmannsreute 46, D-7000 Stuttgart 1 (DE)**

## Beschreibung

Die Erfindung bezieht sich auf ein Einhand-Hysteroskop nach dem Oberbegriff des Anspruchs 1.

Bei der Inspektion und operativen Behandlung des Cervix-Kanales und des Cavum uteri durch ein Hysteroskop ist die Einhandbedienung dieses Hysteroskopes üblich. Hierbei ist vorgesehen, daß das Sehrohr gegen den Druck einer Feder in das Cavum uteri vorgeschoben wird (vgl. bspw. FR-A- 2 520 223). Dadurch ergibt sich aber der schwere Nachteil, daß die unter Längsdruck stehende Optik ständig durch den Daumen gegen den Federdruck in der gewünschten Position gehalten werden muß, damit die endoskopische Orientierung erhalten bleibt. Dies ist besonders kritisch bei der Gewebeentnahme mit Hilfe einer Biopsie-Zange unter optischer Sicht. Hierzu ist weiter bekannt, ein Hilfsinstrument wie eine Biopsie-Zange in dem Schaft unterzubringen, um eine Gewebeprobe zu entnehmen. Durch die Betätigung der Biopsie-Zange wird dabei das patientennahe Ende des Hilfsinstrumentes in Drehung versetzt.

Die Erfindung geht von der Erkenntnis aus, daß von großer Bedeutung ist, daß die Optik des Hysteroskopes bei einer Biopsie präzise in der gewünschten Stellung verbleibt.

Der Erfindung liegt die Aufgabe zugrunde, das Einhand-Hysteroskop so zu verbessern, daß die Betätigung wesentlich erleichtert ist. Insbesondere soll ein schrittweises Vorschieben der Optik sowie eine Arretierung derselben in jeder beliebigen Stellung automatisch erfolgen, ohne daß eine bestimmte Handkraft aufrecht erhalten bleiben muß.

Hierbei soll auch möglich sein, Hysteroskop-Schäfte mit verschiedenen Durchmessern zu verwenden.

Zur Lösung dieser Aufgabe sind die kennzeichnenden Merkmale des Anspruchs 1 vorgesehen. Auf diese Weise muß die Handkraft nur dann angewendet werden, wenn das schrittweise Vorschieben der Optik unter Sichtbeobachtung erfolgt.

Durch die kennzeichnenden Merkmale der Ansprüche 4 bis 7 wird ferner erreicht, daß Hyteroskop-Schäfte mit unterschiedlicher Größe für die Untersuchung und die Operation ausgewechselt werden können.

Weitere Vorteile und Einzelheiten der Erfindung ergeben sich aus der nun folgenden Beschreibung eines Ausführungsbeispiels unter Hinweis auf die Zeichnung. In dieser zeigen:

Fig. 1 eine Seitenansicht auf das gesamte Einhand-Hysteroskop mit gelöster Dichtungs-Verschraubung;

Fig. 2 eine Seitenansicht auf eine Einzelheit nach der Fig. 1 ;

Fig. 3 die Dichtungskappe nach Fig. 1 für sich allein;

Fig. 4 die Gummi-Überwurfshülse nach der Fig. 1 für sich allein und

Fig. 5 eine Teilansicht auf das Instrument nach Fig. 1 in stark vergrößerndem Maßstab.

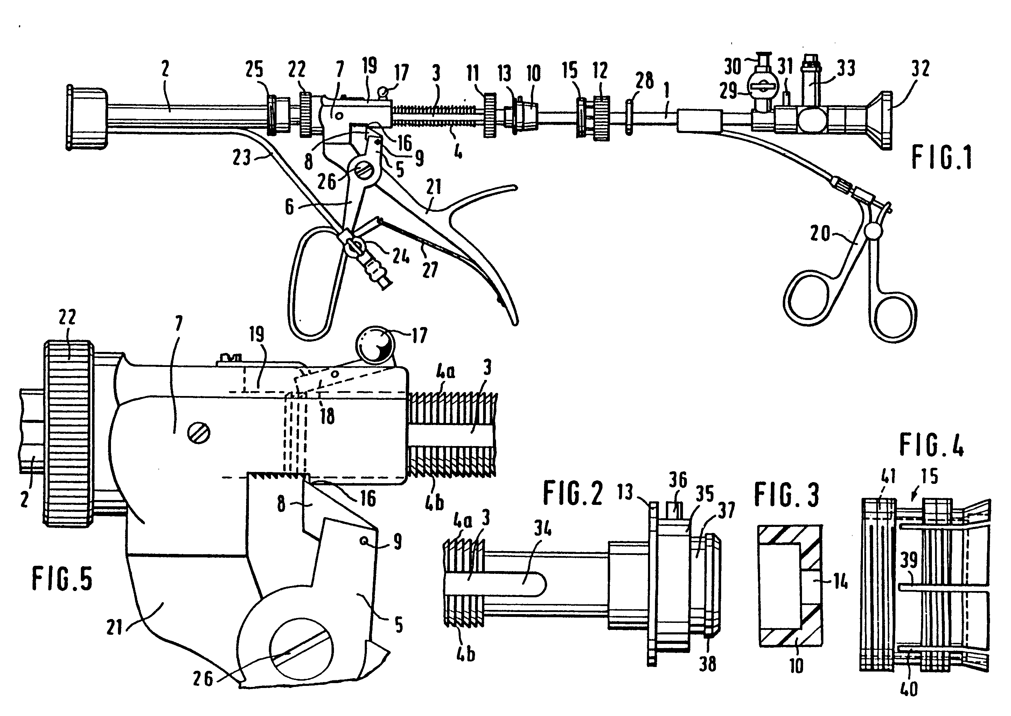

Das Einhand-Hysteroskop nach Fig. 1 zeigt links am patientennahen Ende den Hysteroskop-Adapter 2 zur Fixierung an der Portio durch Vakuum. Dieses Vakuum wird durch einen Vakuum-Kanal hergestellt, der über den Hahn 24 zur Pumpe oder Spritze für die Vakuum-Fixierung angeschlossen wird. Es können mehrere Größen dieses Adapters mit verschiedenen Bohrungen mit einem Untersuchungs- oder einem Operationsschaft 1 Verwendung finden. Weiter rechts sieht man an dem Adapter 2 einen Gewindebund 25, der zur Befestigung mittels der Überwurfmutter 22 an dem Hülsenlager 7 dient.

Mit dem Hülsenlager 7 fest verbunden ist unten die starre Zangenhälfte 21 sichtbar, um die die Zangenhälfte 6 um die Schraube 26 schwenkbar ist. Zwischen den beiden Zangenhälften 21 und 6 befindet sich eine Federeinrichtung 27 bekannter Bauart, welche die Zangenhälften bei Nichtgebrauch auseinanderschiebt.

Über der Schaftlagerung 19 sieht man einen Knopf 17, der zur Betätigung eines hier nicht sichtbaren Sperrhebels 18 dient, der in Fig. 5 dargestellt ist.

Darunter ist ein bewegliches Zangenmaulteil 5 erkennbar, welches mit einem beweglichen Eingriffselement 8 versehen ist, wie später noch erläutert wird.

Aus dem Lager 19 ragt nach rechts die Betätigungshülse 3, auf der eine Überwurfmutter 11 lose dargestellt ist, die mit einer Rändelung versehen ist. Weiter rechts sieht man eine Stufenscheibe 13, die mit der Betätigungshülse 3 fest verbunden ist und die Gummidichtung 10 aufnimmt, wie später noch erläutert wird. Weiter rechts ist die Gummi-Überwurfhülse 12 zusammen mit einer weiteren Überwurfmutter 15 sichtbar, die zum Verschrauben der Dichtung 10 bestimmt ist. Dies gilt auch für die Scheibe 28, die weiter rechts auf dem Schaft 1 dargestellt ist.

Schließlich sieht man am patientenfernen Ende des Endoskopschaftes 1 den üblichen Anschluß 30 mit einem Hahn 29 zur Gasinsufflation oder Instillation von Flüssigkeiten.

Ganz rechts ist schließlich der Verschluß 31 für die Optik vorhanden, deren Okular 32 sichtbar ist, während oben der Anschluß 33 für den Lichtleiter vorgesehen ist.

Hysteroskope dieser allgemeinen Art sind dem Fachmann bekannt und müssen daher nicht in Einzelheiten geschildert werden. Im nachfolgenden werden deshalb nur diejenigen Teile im einzelnen erläutert, die gegenüber dem Stand der Technik neu und erfindungswesentlich sind.

Fig. 2 zeigt nur einen Teil der Betätigungshülse 3 nach der Fig. 1, die in dem Lager 9 zur Verschiebung des Endoskopschaftes 1 vorgesehen ist.

Die Hülse 3 zeigt oben sägezahnförmige Kerben 4 a und unten die gleichen Kerben 4 b, welche durch eine Führungsnut 34 voneinander getrennt sind. Weiter rechts ist die Stufenscheibe 13 sichtbar, deren linker Durchmesser maximal ist. Daran schließt sich rechts ein Teil der Scheibe mit einem kleineren Durchmesser 35 an, der oben mit einem Stift 36 versehen ist. Daran schließt sich rechts ein Teil 37 mit einem nochkleineren Durchmesser an, der ganz rechts eine Wulst 38 aufweist.

Fig. 3 zeigt eine hutförmige Dichtungskappe 10 im Schnitt, die auf das Teil 37 aufsetzbar ist. Es zeigt sich ein Mittenloch 14, das zum Durchgang des Endoskopschaftes einen wesentlich kleineren Durchmesser hat als dieser.

Fig. 4 zeigt eine Gummi-Überwurfhülse 15 mit einigen Längsschlitzen 39, 40, außerdem ist eine Nut 41 zurAufnahme des Stiftes 36 vorgesehen. Die Schlitze 39 , 40 sind vorgesehen, damit die Gummi-Überwurfhülse noch elastischer ist als andernfalls. Diese Hülse 15 wird über die Gummidichtung 10 geschoben, bis sie an die Scheibe 13 anschlägt.

Fig. 5 zeigt das Hülsenlager 7 mit der schon erwähnten Betätigungshülse 3, die zur Aufnahme des Endoskopschaftes 1 dient, der hier jedoch nicht dargestellt ist. Links sieht man wieder den Adapter 2, der mittels der mit einer Rändelung versehenen Überwurfmutter 22 an das Hülsenlager 7 geschraubt ist. Darunter ist ein Teil der feststehenden Zangenhälfte 21 sichtbar, in der mittels der Schraube 26 die bewegbare Zangenhälfte 6 gemäß der Fig. 1 befestigt ist. Hier ist nun Zangengriffraste 5 deutlich zu sehen, das oben rechts mit einem federbelasteten Eingriffselement 8 versehen ist, welches um das Gelenk 9 schwenkbar ist. Die Feder zur Beaufschlagung des Eingriffelementes ist hier nicht dargestellt, sie ist in der Zangengriffraste (5) angeordnet.

Hier ist auch deutlich zu erkennen, daß das Hülsenlager 7 mit einem Anschlag 16 versehen ist, gegen den das Eingriffselement derart anschlägt, daß es in dieser Ruhelage nicht in die Kerben 4 b eingreift. Dies ist wichtig, damit in dieser Stellung die Betätigungshülse 3 nach rechts zurückgezogen werden kann, wenn der Knopf 17 oben an dem Hülsenlager 7 nach unten gedrückt wird. Dadurch wird nämlich der federbelastete Sperrhebel 18 um sein Schwenklager 19 aus dem Eingriff mit den Kerben 4 a herausgeschwenkt. Der Sperrhebel 18 selbst ist nur durch unterbrochene Linien dargestellt, weil er hier sonst nicht sichtbar ist. Die Feder zur Rückstellung des Hebels in seine dargestellte Sperrlage ist hier nicht dargestellt. Der Fachmann kann sich leicht eine Blattfeder hierzu vorstellen.

Im nachfolgenden wird die Wirkungsweise des Erfindungsgegenstandes erläutert. In der in den Fig. 1 und 5 dargestellten Ruhelage werden die beiden Zangengriffhälften 6 und 21 durch die Feder 27 auseinander gedrückt, so daß das Eingriffselement 8 sich außerhalb des Eingriffes mit den sägezahnförmigen Kerben 4 b befindet. Wenn nun der Arzt mit einer Hand die bewegliche Zangengriffhälfte 6 betätigt, wird die Zangengriffraste 5 nach links gedrückt und kommt mit den sägezahnförmigen Kerben 4 b in Eingriff. Bei weiterer Betätigung wird dadurch die Betätigungsstange 3 schrittweise nach links zusammen mit dem Schaft 1 gedrückt. Dabei rastet der Sperrhebel 18 in die sägezahnförmigen Kerben 4 a federbelastet ein, so daß ein Zurückgleiten, der Betätigungshülse 3 nicht möglich ist. Dagegen kann aber die Zangengriffhälfte 6 durch Betätigung der Federeinrichtung 27 wieder zurückgelangen, weil nämlich das schwenkbare und federbelastete Eingriffselement 8 nachgibt und über die Kerbe 4 b zurückgleitet. Durch Betätigung der Zange ist somit ein schrittweises Vorschieben der Betätigungshülse 3 und damit des Schaftes 1 möglich, wobei durch den Sperrhebel eine Arretierung nach rückwärts erfolgt.

Dadurch kann der Arzt ohne Betätigung des Instrumentes sicher sein, daß der Endoskopschaft mit dem Sehrohr nicht zurückgleitet, wenn keine Handkraft ausgeübt wird.

Soll nun der Endoskopschaft zurückgezogen werden, so ist lediglich erforderlich, den Knopf 17 nach unten zu drücken, wonach die Hülse 3 leicht nach hinten nachgibt und von Hand bis zu einem Anschlag herausgezogen werden kann.

Während der Beobachtung kann auch das Hilfsinstrument 20 in bekannter Weise betätigt werden, was durch die Erfindung umso leichter ist, weil der Arzt während des Stillstandes des Endoskopschaftes keine Handkraft ausüben muß. Zum Beispiel kann durch die dargestellte Biopsie-Zange 20 Gewebe von der beobachteten Stelle entnommen werden.

Ein weiterer durch die Erfindung erzielbarer Vorteil besteht darin, daß in der erfindungsgemäßen Betätigungshülse 3 durch die erwähnte Dichtung nach den Fig. 2-4 Hysteroskopschäfte unterschiedlicher Größe für die Untersuchung oder die Operation ausgewechselt werden können.

**Patentansprüche**

1. Einhand-Hysteroskop mit einem Hysteroskop-Adapter (2) zur Fixierung an der Portio durch Vakuum, bei dem der Hysteroskop-Schaft (1) mit dem Sehrohr gegenüber dem Adapter während der Hysteroskopie in die Körperhöhle des Patienten durch Einhand-Betätigung vorschiebbar ist, dadurch gekennzeichnet, daß zur Lagerung des Hysteroskop-Schaftes (1) in dem Hysteroskop-Adaper (2) eine Betätigungshülse (3) vorgesehen ist, die zum schrittweisen Vorschieben außen mit Kerben (4) zum Eingriff einer beweglichen Zangengriffraste (5) einer Zangengriffhälfte (6) versehen ist, welche an dem feststehenden Hülsengrifflager (7) zusammen mit einer feststehenden Zangenhälfte (21) angeordnet ist.

2. Einhand-Hysteroskop nach Anspruch 1, dadurch gekennzeichnet, daß die Kerben (4) sägezahnförmig ausgebildet sind.

3. Einhand-Hysteroskop nach Anspruch 1, dadurch gekennzeichnet, daß die bewegliche Zangengriffraste (5) mit einem federbelasteten um ein Gelenk (9) schwenkbaren Griffelement (8) versehen ist.

4. Einhand-Hysteroskop nach Anspruch 1, dadurch gekennzeichnet, daß die Betätigungshülse (3) an ihrem patientenfernen Ende eine Gummidichtung (10) aufweist, so daß Hysteroskop-Schäfte (1) unterschiedlicher Grösse verwendet werden können.

5. Einhand-Hysteroskop nach Anspruch 4, dadurch gekennzeichnet, daß die Gummidichtung (10) zwischen 2 Überwurfmuttern (11,12) mit einer Außen-Rändelung angeordnet ist und daß die Betätigungshülse (3) mit einer Stufenscheibe (13) zur Aufnahme der Gummidichtung (10) versehen ist.

6. Einhand-Hysteroskop nach Anspruch 5, dadurch gekennzeichnet, daß die Gummidichtung (10) hutförmig auf der Betätigungshülse (3) sitzt und eine zentrale Öffnung (14) zum Durchgang des Schaftes (1) aufweist.

7. Einhand-Hysteroskop nach Anspruch 6, dadurch gekennzeichnet, daß eine gegen Verdrehung gesicherte Gummihülse (15) auf der hutförmigen Gummidichtung (10) sitzt.

8. Einhand-Hysteroskop nach Anspruch 3, dadurch gekennzeichnet, daß ein Anschlag (16) an dem Hülsenlager (7) angeordnet ist, so daß das Eingriffselement (8) in seiner Nichtgebrauchslage außer Eingriff mit den Kerben (4) steht und daß eine federbelastete Sperre (17,18) in die Kerben (4) eingreift.

9. Einhand-Hysteroskop nach Anspruch 8, dadurch gekennzeichnet, daß die Sperre einen federbelasteten Sperrhebel (18) aufweist.

10. Einhand-Hysteroskop nach Anspruch 1, dadurch gekennzeichnet, daß in dem Hysteroskop-Schaft (1) ein Hilfsinstrument (20) angeordnet ist.

## Claims

1. One-hand hysteroscope with a hysteroscope adapter (2) for fixing to the uterine neck by vacuum, in which by one-hand operation the hysteroscope shank (1) with the viewing tube can be advanced with respect to the adapter into the body cavity of the patient during hysteroscopy, characterized in that an actuating sleeve (3) is provided for mounting the hysteroscope shank (1) in the hysteroscope adapter (2) and which, for stepwise advance, is externally provided with notches (4) for engaging a movable forcep detent (5) of a forcep half (6), which is arranged on the fixed sleeve grip bearing (7) together with a fixed forcep half (21).

2. One-hand hysteroscope according to claim 1, characterized in that the notches (4) are constructed in sawtooth-like manner.

3. One-hand hysteroscope according to claim 1, characterized in that the movable forcep detent (5) is provided with a spring-loaded gripping member (8) pivotable about a joint (9).

4. One-hand hysteroscope according to claim 1, characterized in that at its end remote from the patient, the actuating sleeve (3) has a rubber gasket (10), so that it is possible to use hysteroscope shanks (1) of different sizes.

5. One-hand hysteroscope according to claim 4, chracterized in that the rubber gasket (10) is located between two box nuts (11, 12) with external knurling and that the actuating sleeve (3) is provided with a step pulley (13) for receiving the rubber gasket (10).

6. One-hand hysteroscope according to claim 5, characterized in that the rubber gasket (10) is positioned in cap-like manner on the actuating sleeve (5) and has a central opening (14) for the passage of shank (1).

7. One-hand hysteroscope according to claim 6, characterized in that a rubber sleeve (13) is positioned on cap-shaped rubber gasket (10) so that it is prevented from twisting.

8. One-hand hysteroscope according to claim 3, characterized in that a stop (16) is provided on the sleeve bearing (7), so that in its non-use position, gripping member (8) is disengaged from notches (4) and that a spring-loaded catch (17, 18) engages in notches (4).

9. One-hand hysteroscope according to claim 8, characterized in that the catch has a spring-loaded pawl (18).

10. One-hand hysteroscope according to claim 1, characterized in that an auxiliary instrument (20) is arranged in hysteroscope shank (1).

## Revendications

1. Hystéroscope à une seule main, comportant un manchon de fixation (2) pour la fixation de l'hystéroscope à la portion du col de l'utérus au moyen de vide dans lequel la tige (1) de l'hystéroscope, comportant le conduit de vision, est capable d'avancer dans la cavité utérine de la patiente, par rapport au manchon de fixation, par commande d'une seule main pendant l'hystéroscopie, caractérisé en ce que, pour le logement de la tige (1) de l'hystéroscope dans le manchon de fixation (2) de l'hystéroscope, est prévu un manchon de commande (3) qui est muni à l'extérieur, pour l'avancement graduel, de crans (4) pour l'engrènement d'une branche d'encliquetage mobile (5) d'une moitié (6) d'une pince d'arrêt, laquelle est disposée sur la pièce fixe de logement (7) du manchon, conjointement avec une moitié de pince fixe (21).

2. Hystéroscope à une seule main selon la revendication 1, caractérisé en ce que les crans (4) sont en forme de dents de scie.

3. Hystéroscope à une seule main selon la revendication 1, caractérisé en ce que la branche d'encliquetage mobile (5) est munie d'un élément de prise (8) actionné par ressort, pivotant autour d'une articulation (9).

4. Hystéroscope à une seule main selon la revendication 1, caractérisé en ce que le manchon de commande (3) présente, à son extrémité opposée à la patiente, un joint de caoutchouc (10), si bien qu'il est possible d'utiliser des tiges d'hystéroscope (1) de différentes tailles.

5. Hystéroscope à une seule main selon la revendication 4, caractérisé en ce que le joint de caoutchouc (10) est disposé entre deux écrous-chapeaux (11, 12) comportant un moletage externe, et en ce que le manchon de commande (3) est muni d'un cône à gradins (13) pour la reception du joint de caoutchouc (10).

6. Hystéroscope à une seule main selon la revendication 5, caractérisé en ce que le joint de caoutchouc (10) repose en forme de chapeau sur le manchon de commande (3) et présente une ouverture centrale (14) pour le passage de la tige (1).

7. Hystéroscope à une seule main selon la revendication 6, caractérisé en ce qu'un manchon de caoutchouc (15) bloqué vis-à-vis de la rotation, repose sur le joint de caoutchouc (10) en forme de chapeau.

8. Hystéroscope à une seule main selon la revendication 3, caractérisé en ce qu'une butée (16) est disposée sur la pièce de logement (7) du manchon, si bien que l'élément de prise (8) reste hors de prise des crans (4) dans sa position de non utilisation, et en ce qu'un cliquet (17, 18) mû par ressort s'accroche dans les crans (4).

9. Hystéroscope à une seule main selon la revendication 8, caractérisé en ce que le cliquet présente un levier d'arrêt (18) actionné par ressort.

10. Hystéroscope à une seule main selon la revendication 1, caractérisé en ce qu'un instrument auxiliaire (20) est disposé dans la tige (1) de l'hystéroscope.

FIG.1

FIG.2 13 36 35 37

FIG.3

FIG.4

FIG.5

0 157 888